# EUROPÄISCHE PATENTANMELDUNG

(11) **EP 3 496 046 A1**
(43) Veröffentlichungstag der Anmeldung: **12.06.2019**
(21) Anmeldenummer: 18156440.2
(22) Anmeldetag: 13.02.2018
(51) Int. Cl.: G06T 19/00, G06T 19/20

(54) **VERFAHREN ZUR DARSTELLUNG DREIDIMENSIONALER MEDIZINISCHER BILDINFORMATIONEN**

(71) Anmelder: Siemens Healthcare GmbH, 91052 Erlangen (DE)
(72) Erfinder: Kuth, Rainer, 91315 Höchstadt (DE); Schmidt, Sebastian, 91085 Weisendorf (DE)

(57) **Zusammenfassung**

Die vorliegende Erfindung betrifft ein Verfahren zur Darstellung von medizinischen Bilddaten auf mindestens einem Anzeigemedium für eine Gruppe aus mindestens zwei interaktiven Betrachtern, mit den Schritten
- Bereitstellen von medizinischen Bilddaten, welche zumindest einen drei- oder vierdimensionalen Bilddatensatz eines bestimmten Untersuchungsbereichs eines Patienten enthalten;
- Bereitstellen einer Möglichkeit zur perspektivischen Darstellung der medizinischen Bilddaten in einer interaktiven, virtuellen Umgebung, insbesondere mit erweiterter Realität;
- wobei jedem interaktiven Betrachter eine eigene virtuelle Position und ggf. ein eigener Blickwinkel in der virtuellen Umgebung zugeordnet ist; und
- wobei jedem der interaktiven Betrachter gleichzeitig die medizinische Bilddaten aus der dem jeweiligen Betrachter zugeordneten virtuellen Position und ggf. Blickwinkel auf dem mindestens einen Anzeigemedium dargestellt wird, und
- wobei jeder interaktiver Betrachter seine virtuelle Position und ggf. Blickwinkel unabhängig von den anderen Betrachten verändern kann. Ferner betrifft die vorliegende Erfindung ein Computerprogramm und eine Vorrichtung zur Darstellung von medizinischen Bilddaten.

## Beschreibung

Die vorliegende Erfindung betrifft ein Verfahren zur Darstellung von medizinischen Bilddaten auf mindestens einem Anzeigemedium für eine Gruppe aus mindestens zwei interaktiven Betrachtern.

Bei vielen medizintechnischen Anwendungen werden von einem Expertenteam, insbesondere einem Ärzteteam, dreidimensionale Daten betrachtet und im Dialog analysiert, um einem Patienten eine Therapieempfehlung geben zu können. Für eine vollständige Betrachtung und ein umfassendes Verständnis eines dreidimensionalen Objektes, wie beispielsweise eines Organes eines Patienten, ist ein interaktives Drehen oder ein virtueller Rundgang durch das Objekt notwendig. Beispielsweise werden bei einer sogenannten Tumorkonferenz radiologische Aufnahmen von einem Patienten interdisziplinär begutachtet, um gemeinsam über das weitere Vorgehen, insbesondere über die Therapieplanung, zu entscheiden. Es ist auch denkbar, dass sich beispielsweise ein chirurgisches Team vor einer anstehenden Operation hinsichtlich des gemeinsamen Vorgehens während der Operation abstimmen.

Bei bereits bekannten VR (virtual reality) und AR (augmented rality) Systemen kann ein einziger Betrachter für alle Betrachter einen einzigen Betrachtungswinkel und eine einzige Betrachtungsposition bestimmen. Die weiteren Betrachter sind hierbei nicht in der Lage, sich selbst virtuell zu bewegen, um einen anderen Betrachtungswinkel oder eine andere Betrachtungsposition einnehmen zu können.

Aus der DE 10 2005 020 871 B4 ist ein Verfahren zur Darstellung von medizinischen Bildinformationen auf einem Anzeigemedium bekannt geworden, bei dem die Position eines einzigen Betrachters in Bezug zu dem Anzeigemedium erfasst wird und in Abhängigkeit der Position des Betrachters verschiedene Überlagerungen von wenigstens zwei medizinischen Datensätzen als Bildinformation dem Betrachter dargestellt werden.

Der vorliegenden Erfindung liegt die Aufgabe zugrunde, ein Verfahren anzugeben, mit welchem Betrachter, insbesondere Ärzte unterschiedlicher Disziplinen, die erfassten medizinischen Datensätze eines Patienten zu ein und demselben Untersuchungsbereich interaktiv analysieren können. Ferner liegt der vorliegenden Erfindung die Aufgabe zugrunde ein Computerprogramm und eine Vorrichtung anzugeben, welches/welche ein solches Verfahren ausführen kann.

Die vorliegenden Aufgaben werden gelöst mit einem Verfahren nach Anspruch 1, einem Computerprogramm nach Anspruch 13 und einer Vorrichtung nach Anspruch 14.

Erfindungsgemäß wird ein Verfahren zur Darstellung von medizinischen Bilddaten auf mindestens einem Anzeigemedium für eine Gruppe aus mindestens zwei interaktiven Betrachtern vorgeschlagen, wobei das Verfahren folgende Schritte umfasst:
- Bereitstellen von medizinischen Bilddaten, welche zumindest einen drei- oder vierdimensionalen Bilddatensatz eines bestimmten Untersuchungsbereichs eines Patienten enthalten;
- Bereitstellen einer Möglichkeit zur perspektivischen Darstellung der medizinischen Bilddaten in einer interaktiven, virtuellen Umgebung, insbesondere mit erweiterter Realität;
- wobei jedem interaktiven Betrachter eine eigene virtuelle Position und ggf. ein eigener Blickwinkel in der virtuellen Umgebung zugeordnet ist/sind; und
- wobei jedem der interaktiven Betrachter gleichzeitig die medizinischen Bilddaten aus der dem jeweiligen Betrachter zugeordneten virtuellen Position und ggf. Blickwinkel auf dem mindestens einen Anzeigemedium dargestellt wird, und
- wobei jeder interaktiver Betrachter seine virtuelle Position und ggf. Blickwinkel unabhängig von den anderen Betrachtern verändern kann.

Erfindungsgemäß wird ein Verfahren vorgeschlagen bei dem mehrere interaktive Betrachter, insbesondere Ärzte unterschiedlicher Disziplinen, gleichzeitig eine Darstellung der medizinischen Bilddaten ein und desselben Untersuchungsbereiches eines Patienten dargestellt wird, um die medizinischen Bilddaten zu analysieren. Die medizinischen Bilddaten beinhalten zumindest einen dreidimensionalen (3D) oder vierdimensionalen (4D) Bilddatensatz, wobei die vierte Dimension die Zeit ist, d.h. ein 4D-Bilddatensatz ist eine Zeitreihe von 3D-Bildern. Die Bilddaten können auch mehrere Bilddatensätze desselben Untersuchungsbereichs enthalten, und ggf. weitere Untersuchungsdaten oder -bilder, auch zweidimensionale Bilder. Enthalten die medizinischen Bilddaten mehrere Bilddatensätze des Untersuchungsbereichs, sind diese vorzugsweise miteinander registriert, so dass sie in der virtuellen Umgebung wahlweise oder miteinander überblendet/überlagert dargestellt werden können, oder der jeweilige Betrachter kann auswählen, welchen Bilddatensatz er betrachten möchte. Beispiele für solche überblendbaren/überlagerbaren Bilddaten sind T1, T2, T2*, Multikern bzw. nicht-Protonen MR Bilder, Ultraschallbilder verschiedener Frequenzen, CT Bilder mit unterschiedlichen Beschleunigungsspannungen oder generell Bilder mit und ohne Kontrastmittel.

Bei dem vorgeschlagenen Verfahren werden die medizinischen Bilddaten des Untersuchungsbereiches einem jeden Betrachter als dreidimensionale virtuelle Umgebung dargestellt. In dieser dreidimensionalen virtuellen Umgebung kann sich jeder der interaktiven Betrachter bewegen und in bevorzugten Ausführungsformen auch die medizinischen Bilddaten verändern. Das Verändern kann beispielsweise ein Segmentieren der medizinischen Bilddaten oder ein teilweises Darstellen der medizinischen Bilddaten in einem halbtransparenten Darstellungsmodus umfassen. Hierbei kann jeder der interaktiven Betrachter zeitgleich mit den anderen interaktiven Betrachter die medizinischen Bilddaten in Hinblick auf seine Spezialisierung in der jeweiligen Disziplin die Bilddaten analysieren bzw. auswerten, um anschließend einen Therapievorschlag zu unterbreiten. Ferner können sich die interaktiven Betrachter in der virtuellen Umgebung, insbesondere in dem Untersuchungsbereich, unabhängig voneinander bewegen.

Insbesondere kann ein Arzt einer Disziplin sich als Betrachter bei dem erfindungsgemäßen Verfahren eine andere Darstellung oder ein andere Auswahl der Bilddaten, welche beispielsweise mindestens zwei unterschiedliche Bilddatensätze des Untersuchungsbereiches enthalten, anzeigen lassen als ein anderer Betrachter bzw. Arzt einer anderen Disziplin. Beispielsweise lässt ein Orthopäde sich nur eine Darstellung der medizinischen Bilddaten anzeigen, aus der sich Bildinformationen nur zum Knochenbau und zum Knorpel ergeben, während ein Gefäßchirurg sich bevorzugt nur eine Segmentierung der Bilddaten, oder einen anderen Bilddatensatz anzeigen lässt, aus welchen sich die Gefäßstruktur des Patienten in dem Untersuchungsbereich ergibt. Folglich kann ein Betrachter, insbesondere ein Arzt einer bestimmten Disziplin, sich eine andere Segmentierung dieses Untersuchungsbereiches anzeigen lassen als ein anderer Betrachter, insbesondere als ein anderer Arzt einer anderen Disziplin. Ferner können die verschiedenen Betrachter unterschiedliche virtuelle Positionen mit ggf. jeweils unterschiedlichen Blickwinkeln in der virtuellen Umgebung des Untersuchungsbereiches einnehmen, wodurch ein Betrachter eine Perspektive einnehmen kann, welche nicht einer Perspektive eines anderen Betrachters entsprechen muss aber entsprechen kann. In einigen Ausführungsformen kann jeder Betrachter der virtuellen Umgebung (VR) eine erweiterte Realität (AR) hinzufügen. Die erweiterte Realität kann beispielsweise das Hinzufügen eines Zeigers in die virtuelle Umgebung umfassen.

Eine Möglichkeit zur perspektiven Darstellung der medizinischen Bilddaten ist beispielsweise durch Wiedergabe dieser medizinischen Daten auf einem Anzeigemedium, insbesondere einem 3D-fähigen Anzeigemedium, gegeben. Beispielsweise ist das Anzeigemedium ein oder mehrere Bildschirme, Monitore, Fernseher, Touchpads oder dergleichen, und die Betrachter tragen z.B. jeweils eine 3D-Brille, die die Wahrnehmung in der virtuellen Umgebung in Virtual Reality ermöglicht, z.B. eine Shutterbrille. Um einen räumlichen Eindruck zu vermitteln, werden dabei zwei Bilder der medizinischen Bilddaten aus unterschiedlichen Perspektiven erzeugt und dargestellt (Stereoprojektion). Die Shutterbrille sorgt dafür, dass das jeweilige Bild dem richtigen Auge zugeführt wird. Dabei kann ein Bildschirm pro Betrachter vorhanden sein, ggf. können auch zwei Betrachter den gleichen Bildschirm nutzen, wenn dieser vier verschiedene Bilder im Wechsel anzeigt und die Shutterbrillen entsprechend eingestellt sind. Es ist ferner denkbar die medizinischen Bilddaten als Hologramm darzustellen.

In Vorteilhaften Ausführungsformen können mit dem vorgeschlagenen Verfahren verschiedene medizinische, insbesondere dreidimensionale, erfasste Datensätze derselben anatomischen Region, d.h. desselben Untersuchungsbereiches, überlagert dargestellt werden und jeder Betrachter hat die Möglichkeit, zwischen diesen Datensätzen zu wählen, d.h. zu segmentieren. Die unterschiedlichen medizinischen Datensätze können beispielsweise T1- und T2-gewichtete MRT-Sequenzen, CT-Datensätze mit unterschiedlichen Photonenenergiespektren oder dergleichen umfassen. Es können ferner mindestens zwei medizinische Datensätze derselben anatomischen Region überlagert dargestellt werden, welche mit unterschiedlichen Modalitäten aufgenommen wurden, wie zum Beispiel mittels CT und PET, MR und PET, SPECT, MEG (Magnetenzephalogramm), EEG (Elektroenzephalogramm).

Bevorzugt wird für zumindest einen, vorzugsweise alle, interaktiven Betrachter eine Möglichkeit zur Bearbeitung der medizinischen Bilddaten und/oder zur Veränderung der Darstellung der medizinischen Bilddaten bereitgestellt. Für eine solche Bearbeitung und/oder Veränderung der medizinischen Bilddaten kann in der virtuellen Umgebung beispielsweise ein virtuelles Bedienelement vorgesehen sein, welches dazu ausgebildet ist, die medizinischen Bilddaten zu verändern und/oder eine erweiterte Realität zu den medizinischen Bilddaten hinzuzufügen.

Gemäß einer bevorzugten Ausführungsform des vorgeschlagenen Verfahrens kann jeder Betrachter auswählen, ob nach einer Bearbeitung der medizinischen Bilddaten und/oder Veränderung der Darstellung der medizinischen Bilddaten den anderen Betrachtern oder einer bestimmten Gruppe der anderen Betrachter in Echtzeit der zumindest eine bearbeitete medizinische Bilddatensatz bzw. die veränderte Darstellung auf dem mindestens einen Anzeigemedium dargestellt wird. Das vorgeschlagene Verfahren weist somit den Vorteil auf, dass verschiedene Betrachter die medizinischen Bilddaten in einer virtuellen Umgebung zunächst betrachten und/oder analysieren können und anschließend in Echtzeit mit anderen Betrachtern ihre Darstellung der medizinischen Bilddaten austauschen können, wobei die Darstellung eines Betrachters insbesondere aus dem virtuellen Betrachtungsort und/oder dem Betrachtungswinkel und/oder den Einstellungen und/oder der Auswahl der ausgewählten Kontraste oder Kontrastmischungen, also den Visualisierungseinstellungen eines Betrachters, besteht. Hierdurch kann folglich ein Betrachter seine Bewertung der medizinischen Bilddaten einem anderen oder allen anderen Betrachter in Echtzeit mitteilen, wodurch dem oder den anderen Betrachter(n) die Bewertung des einen Betrachters einfacher erklärt werden kann. Das vorgeschlagene Verfahren hat somit ferner den Vorteil, dass eine Bewertung bzw. Diagnose eines Betrachters den jeweiligen anderen Betrachtern schnell und unkompliziert übermittelt werden kann.

Bevorzugt umfasst die Bearbeitung der medizinischen Bilddaten bzw. die Veränderung der Darstellung der medizinischen Bilddaten mindestens eine der folgenden Aktionen:
- Anbringen eines virtuellen Zeigers, insbesondere wahlweise mit einem hinterlegtem Hyperlink auf einen eingegebenen Text und/oder Grafik und/oder einer Audiospur an einer bestimmten Position eines medizinischen Bilddatensatzes,
- Auswählen einer Segmentierung eines medizinischen Bilddatensatzes, welche beispielsweise nur Knochen und/oder nur Knorpel und/oder nur Gefäße des Untersuchungsbereiches zeigt,
- Umschalten zwischen verschiedenen medizinischen Bilddatensätzen desselben Untersuchungsbereichs,
- Entfernen und/oder Schwärzen von Teilen eines dreidimensionalen medizinischen Bilddatensatzes,
- Überlagern eines ersten dreidimensionalen medizinischen Bilddatensatzes des Untersuchungsbereichs mit einem zweiten dreidimensionalen medizinischen Bilddatensatz des Untersuchungsbereichs, welche insbesondere mit unterschiedlichen Modalitäten oder Gewichtungen oder zu unterschiedlichen Zeitpunkten aufgenommen wurden,
- Einblenden von nicht-ortskodierten Daten des Patienten in die virtuelle Umgebung,
- im Fall von einem vierdimensionalen medizinischen Bilddatensatz, welcher in einer Schleife als 3D-Film dargestellt wird, Anhalten des 3D-Films an einer beliebigen zeitlichen Position,
- Anzeigen eines Laborwertes zu einem dargestellten Organ im Untersuchungsbereich bei einem Anklicken dieses Organes im virtuellen Raum,
- Einblenden funktioneller medizinischer Daten wie beispielsweise medizinische Daten der funktionellen MRT oder der funktionellen CT.

Das Anbringen eines virtuellen Zeigers oder das Einblenden von nicht-ortskodierten Daten des Patienten oder das Anzeigen eines Laborwertes zu einem dargestellten Organ sind beispielsweise Aktionen, welche der erweiterten Realität (AR) zuzuschreiben sind. Hingegen können das Auswählen einer Segmentierung, das Umschalten zwischen verschiedenen medizinischen Bilddatensätzen desselben Untersuchungsbereichs, das Entfernen und/oder Schwärzen von Teilen eines dreidimensionalen medizinischen Bilddatensatzes, oder das Überlagern eines ersten dreidimensionalen medizinischen Bilddatensatzes des Untersuchungsbereichs mit einem zweiten dreidimensionalen medizinischen Bilddatensatz des Untersuchungsbereichs, oder das Abspielen eines 3D-Filmes der virtuellen Umgebung (VR) zugeordnet werden. Beispielsweise können 3D-Datensätze mit einer Zeitauflösung vom atmenden Thorax eines Patienten in einer Schleife als 3D-Film in der virtuellen Umgebung dargestellt werden. Es ist ferner denkbar, dass neben dem Entfernen und/oder Schwärzen von Teilen eines dreidimensionalen medizinischen Bilddatensatzes, zumindest ein Teil des dreidimensionalen medizinischen Bilddatensatzes in einem halbtransparenten oder farblich transparenten Anzeigemodus dargestellt wird.

Unter dem Einblenden funktioneller medizinischer Daten wie beispielsweise den medizinischen Daten der funktionellen MRT versteht man beispielsweise eine Darstellung eines MRT-Signals, welches den BOLD-Effekt nutzt und somit die Hirnaktivität eines Patienten in der virtuellen Umgebung zeigt.

In anderen Ausführungsformen können auch vierdimensionale medizinische Daten wiedergegeben werden, wobei eine Dimension der Zeit zugordnet ist, während die anderen drei Dimension den Ortskoordinaten des Untersuchungsbereiches zugeordnet sind. Vierdimensionale medizinische Daten sind in der virtuellen Umgebung als 3D-Film abspielbar sind. Das Darstellen der medizinischen Bilddaten in einer virtuellen Umgebung (VR) und/oder einer erweiterten Realität (AR) bei dem vorgeschlagenen Verfahren weist den Vorteil auf, dass die medizinischen Bilddaten Betrachter spezifisch bearbeitet bzw. verändert werden können, so dass insbesondere ein Betrachter schnell zu einer Analyse bzw. Diagnose zu den medizinischen Bilddaten gelangen kann.

Bevorzugt wird die medizinische Bildinformation für die Gruppe aus Betrachtern jeweils auf demselben Anzeigemedium oder auf einem für einen Teil der Gruppe aus Betrachtern jeweils eigenem Anzeigemedium dargestellt. Die medizinischen Bilddaten, welche die dargestellte medizinische Bildinformation bilden, werden beispielsweise auf einem Zentralrechner oder einer Cloud gespeichert, so dass jeder der interaktiven Betrachter auf die medizinischen Bilddaten zugreifen kann und sich auf einem Anzeigemedium, wie einem Bildschirm oder in einer holografischen Darstellung, anzeigen lassen kann. Bei dem vorgeschlagenen Verfahren können die medizinischen Bilddaten folglich zentral an einem Ort oder in einer Cloud gespeichert sein, so dass die verschiedenen interaktiven Betrachter auf die medizinischen Bilddaten und/oder die Visualisierungseinstellungen eines anderen zugreifen können. Die Darstellung der medizinischen Bilddaten kann dann jeweils vor Ort bei dem entsprechenden interaktiven Betrachter erfolgen, d.h. auf einem Anzeigemedium des entsprechenden Betrachters. Anschaulich bedeutet dies, dass die medizinischen Bilddaten beispielsweise an einem Ort X gespeichert sein können und mindestens ein Betrachter Kilometer entfernt am Ort Y sich die medizinischen Bilddaten auf einem entsprechenden Anzeigemedium anzeigen lassen kann. Diese Art des Remote Controls in dem vorgeschlagenen Verfahren weist den Vorteil auf, dass für die Ausübung des vorgeschlagenen Verfahrens die einzelnen Betrachter nicht an demselben Ort sein müssen, um das Verfahren, insbesondere gemeinsam, ausführen zu können.

Bevorzugt enthalten die medizinischen Bilddaten mindestens zwei drei- oder vierdimensionale medizinische Bilddatensätze desselben Untersuchungsbereiches, sowie insbesondere weitere nicht-ortskodierte Daten des Patienten. Die mindestens zwei drei- oder vierdimensionalen medizinischen Bilddatensätze desselben Untersuchungsbereiches können überlagert zueinander dargestellt werden. Hierdurch können beispielsweise ein medizinischer Bilddatensatz, welcher mit einer Modalität erfasst wurde, zusammen mit einem anderen medizinischen Bilddatensatz, welcher mit einer anderen medizinischen Modalität erfasst wurde, überlagert dargestellt werden, sofern die mindestens zwei medizinischen Bilddatensätze denselben Untersuchungsbereich, insbesondere desselben Patienten, zeigen.

Bevorzugt werden die medizinischen Bilddaten auf einem Zentralrechner oder in einer Cloud bereitgestellt. Dies hat den Vorteil, dass sich die unterschiedlichen Betrachter an unterschiedlichen Orten aufhalten können und dennoch gemeinsam durch Ausführung des vorgeschlagenen Verfahrens jeweils unabhängig voneinander eine Veränderung der medizinischen Bilddaten vornehmen können, um eine Therapieplanung auf Basis der medizinischen Bilddaten vornehmen können, insbesondere unter Berücksichtigung verschiedener fachlicher Ausrichtungen der verschiedenen interaktiven Betrachter.

Bevorzugt wird die virtuelle, interaktive Umgebung für jeden Betrachter durch einen 3D-fähigen virtuellen oder realen Client aufgebaut wird. Unter einem virtuellen Client ist vorliegend ein Computerprogramm zu verstehen, welches auf einem Endgerät ausgeführt wird und in einem Netzwerk eingebunden ist und mit einem Zentralrechner bidirektional kommuniziert. Alternativ ist vorliegend ferner unter einem virtuellen Client eine Anzeige eines virtuellen Desktop auf einem Betrachter seitigen Endgerät wie einem Computer, einem iPad oder einem sonstigen bekannten Endgerät zu verstehen, wobei die Daten zur Anzeige des virtuellen Desktops und/oder der medizinischen Bilddaten als "Visualisierungseinstellungen" auf einem Zentralrechner gespeichert sind. Die Kommunikation zwischen dem Endgerät und dem Zentralrechner erfolgt bevorzugt über eine Internetverbindung oder über eine VPN-Verbindung. Bei Verwendung eines virtuellen Clients werden die veränderten medizinischen Bilddaten dem jeweiligen interaktiven Betrachter zugeordnet, welcher die Veränderung vornimmt bzw. vorgenommen hat, und beim Speichern auf dem Zentralrechner gespeichert. Dies hat den Vorteil, dass ein und derselbe interaktive Betrachter bei Verwendung eines anderen Endgerätes Zugriff auf die Veränderung hat. Unter einem realen Client ist vorliegend das Endgerät als solches zu verstehen, welches eingebunden in einem Netzwerk mit einem Zentralrechner bidirektional kommuniziert. Hier kann eine vorgenommene Veränderung auf dem Endgerät ausschließlich oder zusätzlich zu der Speicherung auf dem Zentralrechner gespeichert werden.

Weiter bevorzugt wird in der virtuellen Umgebung einem ersten interaktiven Betrachter mindestens eine Grafikfigur an einer Position dargestellt, welcher der virtuellen Position eines anderen Betrachters in der virtuellen Umgebung entspricht, wobei aufgrund der Position der Grafikfigur dem ersten Betrachter der Gruppe die virtuelle Position des jeweiligen anderen Betrachters in der Darstellung der medizinischen Bilddaten mitgeteilt wird. Ferner ist es denkbar, dem ersten interaktiven Betrachter seine ihm zugeordnete Grafikfigur in der virtuellen Umgebung entweder einzublenden oder auszublenden. Beispielsweise kann der erste interaktive Betrachter bei Bedarf seine ihm zugeordnete Grafikfigur, insbesondere mittels eines Bedienelementes wie der 3D Maus, ein- oder ausblenden lassen. Hierdurch kann der erste interaktive Betrachter seine eigne virtuelle Position bzw. seinen Blickwinkel mit den Positionen bzw. Blickwinkeln der anderen interaktiven Betrachtern vergleichen bzw. abgleichen.

Weiter bevorzugt kann jeder Betrachter die Position und/oder den Blickwinkel eines anderen Betrachters einnehmen, oder ein Betrachter kann veranlassen, dass ein anderer Betrachter in der virtuellen Umgebung seine Position und/oder seinen Blickwinkel einnimmt. Hierdurch kann ein Betrachter oder können mehrere Betrachter einfach und schnell die Position bzw. den Blickwinkel eines anderen Betrachters einnehmen, um dessen Analyse bzw. Bewertung der medizinischen Bilddaten einfacher nachvollziehen zu können. Es ist denkbar, dass ein Betrachter die anderen Betrachter veranlasst seine Position einzunehmen, ohne dass die anderen Betrachter eine Position wählen können.

Bevorzugt erfolgt ein Umschalten von einer Position eines Betrachters auf eine andere Position, insbesondere auf eine Position eines anderen Betrachters, mittels eines allmählichen Perspektivwechsels. Bei einem allmählichen Perspektivwechsel können sogenannte "weiche Trajektorien" der interaktiven Betrachter berechnet werden. Für die Berechnung der weichen Trajektorien wird die Position der einem jeden der interaktiven Betrachter zugeordneten Grafikfigur in der virtuellen Umgebung berücksichtigt. Hierdurch kann ein abrupter Perspektivenwechsel vermieden werden, welche für den einzelnen Betrachter verwirrend sein kann.

Bevorzugt ist zur Ausführung des vorgeschlagenen Verfahrens für jeden Betrachter ein virtuelles Bedienelement in der virtuellen Umgebung angeordnet. Das virtuelle Bedienelement kann in der virtuellen Umgebung beispielsweise als 3D-Maus oder Joystick oder als virtuelle Tastatur oder als virtuelle Konsole, insbesondere neben einem Patientendatensatz, abgebildet sein. Mit dem virtuellen Bedienelement kann ein interaktiver Betrachter jedenfalls die virtuelle Umgebung verändern, wie hierin bereits beschrieben, nämlich beispielsweise durch Hinzufügen eines Zeigers oder durch Segmentieren der medizinischen Bilddaten etc.

Ein weiterer Aspekt der vorliegenden Erfindung betrifft ein Computerprogramm, enthaltend Softwarecode-Abschnitte, wobei die Softwarecode-Abschnitte einen Computer oder ein Netzwerk aus Rechnern oder ein Endgerät in einem Netzwerk, welches mit einem Zentralrechner kommuniziert, dazu veranlassen, ein Verfahren wie hierin beschrieben auszuführen.

Ein weiterer Aspekt der vorliegenden Erfindung betrifft eine Vorrichtung zur Darstellung von medizinischen Bilddaten auf mindestens einem Anzeigemedium für eine Gruppe aus mindestens zwei interaktiven Betrachtern, wobei die Vorrichtung insbesondere zur Durchführung des Verfahrens wie hierin beschrieben konfiguriert ist, umfassend
- mindestens ein Speichermedium zum Hinterlegen der medizinischen Bilddaten, wobei die Bilddaten mindestens einen drei- oder vierdimensionale Bilddatensatz eines bestimmten Untersuchungsbereichs eines Patienten enthalten,
- mindesten ein Anzeigemedium, welches für eine perspektivische dreidimensionale Darstellung der medizinischen Bilddaten geeignet ist,
- eine Recheneinheit, welche dazu konfiguriert ist, die medizinischen Bilddaten für einen interaktiven Betrachter in einer interaktiven, virtuellen Umgebung perspektivisch darzustellen, insbesondere mit erweiterter Realität;
wobei jedem interaktiven Betrachter eine eigene virtuelle Position und ggf. ein eigener Blickwinkel in der virtuellen Umgebung zugeordnet ist; und
wobei jedem der interaktiven Betrachter gleichzeitig die medizinische Bilddaten aus der dem jeweiligen Betrachter zugeordneten virtuellen Position und ggf. Blickwinkel auf dem mindestens einen Anzeigemedium dargestellt werden, und
- wobei jeder interaktiver Betrachter seine virtuelle Position und ggf. Blickwinkel unabhängig von den anderen Betrachten verändern kann.

Bevorzugt umfasst die Vorrichtung ferner eine dreidimensionale Maus für mindestens einen Betrachter und/oder eine 3D-Brille für jeden Betrachter. Besonders bevorzugt umfasst die Vorrichtung für jeden Betrachter eine dreidimensionale Maus, so dass jeder der interaktiven Betrachter die Darstellung der medizinischen Bilddaten in seiner virtuellen Umgebung verändern bzw. bearbeiten kann. Ferner bevorzugt umfasst die Vorrichtung für jeden der interaktiven Betrachter eine 3D-Brille, welche dazu ausgebildet ist, die medizinischen Bilddaten als medizinische Bildinformation den einzelnen Betrachtern darzustellen. Es ist ferner denkbar, dass die Vorrichtung anstatt oder zusätzlich zu einer dreidimensionalen Maus, medizinische Operationswerkzeuge umfasst, mit welchen beispielsweise eine anstehende Operation an dem Untersuchungsbereich simuliert werden kann.

Weitere Vorteile und Merkmale der vorliegenden Erfindung ergeben sich aus der nachfolgenden Beschreibung bevorzugter Ausführungsformen mit Bezug auf die beigefügten Figuren. Es versteht sich, dass einzelne, in den jeweiligen Figuren gezeigte, Ausführungsformen Merkmale aufweisen können, die auch in anderen Ausführungsformen zum Einsatz gelangen können, auch wenn dies nicht explizit genannt ist, und sofern dies nicht aufgrund technischer Gegebenheiten oder explizit ausgeschlossen wurde. Es zeigen:
- Fig. 1: eine Vorrichtung zum Messen von medizinischen Bilddaten, welche in einem Zentralrechnergespeichert werden und an zwei Anzeigemedien angezeigt werden,
- Fig. 2: mehrere interaktive Betrachter an verschiedenen Orten, welche auf die erfassten medizinischen Bilddaten zugreifen, und
- Fig. 3: ein Anzeigemedium, auf dem eine virtuelle Umgebung und eine erweiterte Realität dargestellt sind.

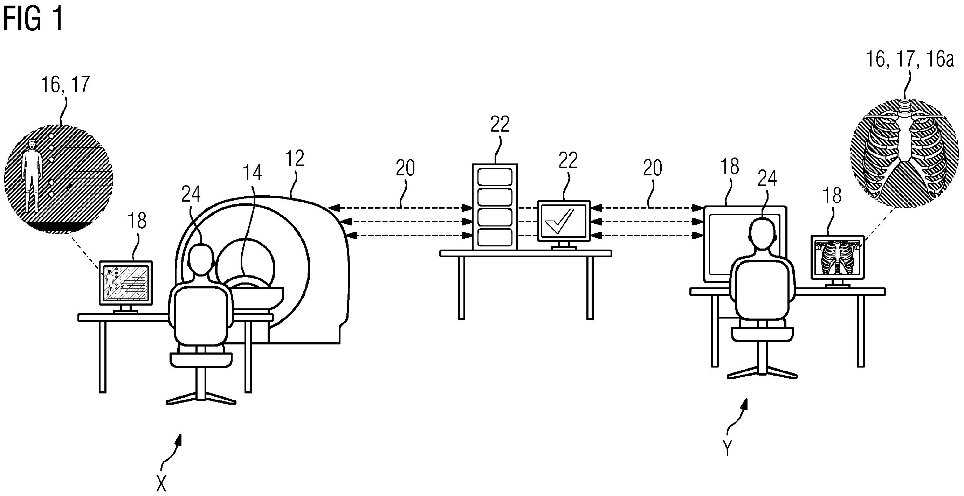

Bevorzugte Ausführungsformen der vorliegenden Erfindung werden nachfolgend in Zusammenschau der Figuren 1 bis 3 beschrieben.

Figur 1 zeigt einen ersten interaktiven Betrachter 24 an einem Ort X, insbesondere einen Arzt oder medizinisches Personal, welcher/welches mittels einer medizinischen Modalität 12 medizinische Bilddaten 16 bzw. medizinische Bilddatensätze 17 eines Patienten 14 erfasst, wobei die medizinischen Bilddaten 16 auf einem Anzeigemedium 18 angezeigt werden. Die gemessenen medizinischen Bilddaten 16 werden mittels bidirektionaler Kommunikation auf einem Zentralrechner 22 gespeichert. Ein anderer interaktiver Betrachter 24 greift mittels bidirektionaler Kommunikation 20 auf die medizinischen Bilddaten 16 bzw. die medizinischen Bilddatensätze 17 zu. Die medizinischen Bilddaten 16 können dabei für den anderen interaktiven Betrachter 24 segmentiert angezeigt werden, so dass der der andere Betrachter 24 einen veränderten medizinischen Bilddatensatz 16a betrachten kann. Der andere Betrachter 24 befindet sich zudem an einem anderen Ort Y, welcher von dem Ort X entfernt gelegen sein kann.

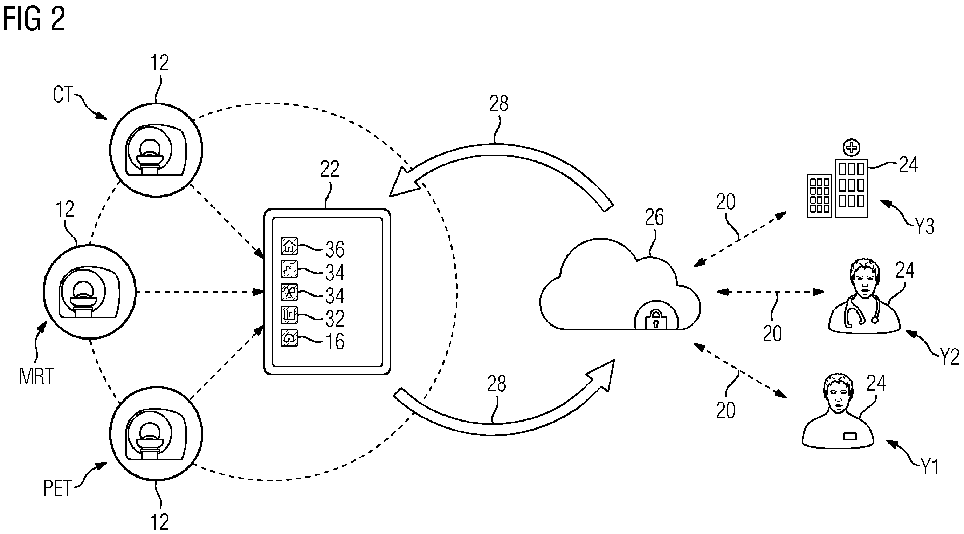

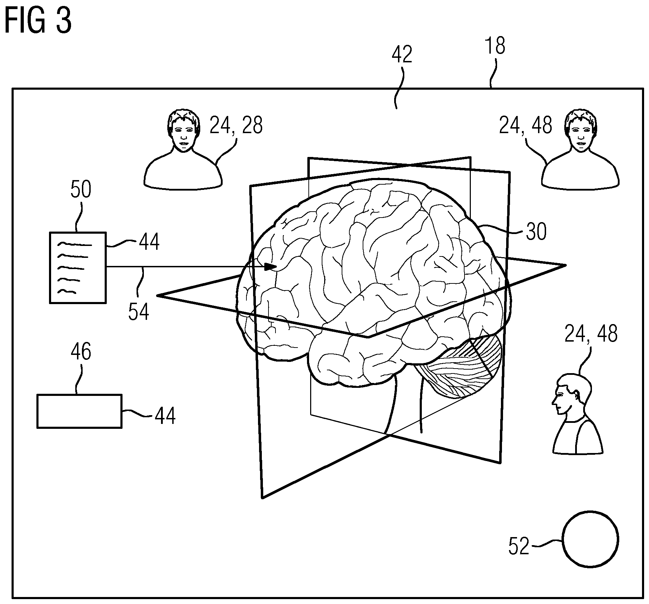

Figur 2 zeigt schematisch, eine mögliche Vorrichtung zur Ausführung des vorgeschlagenen Verfahrens. Mittels verschiedener medizinischer Modalitäten 12, wie beispielsweise einem CT, einem MRT, einem PET, einem SPECT, einem MEG (Magnetenzephalogramm) oder einem EEG (Elektroenzephalogramm,) werden medizinische Bilddaten 16 bzw. medizinische Bilddatensätze 17 ein und desselben Untersuchungsbereiches desselben Patienten 14 gemessen und einem Zentralrechner 22 übergeben, auf welchem die medizinmischen Bilddaten 16 bzw. die medizinischen Bilddatensätze 17 gespeichert werden. Die medizinischen Bilddaten 16 werden vom Zentralrechner 22 einer Cloud 26 über eine bidirektionale Kommunikation 28 bereitgestellt. Die interaktiven Betrachter 24, welche sich an verschiedenen Orten Y1, Y2, Y3 befinden können, können dann mittels einer bidirektionalen Kommunikation 20 auf die medizinischen Bilddaten 16 unabhängig voneinander zugreifen. Neben den medizinischen Bilddaten 16 werden auf dem Zentralrechner 22 bzw. in der Cloud 26 ferner beispielsweise Messprotokolle 32, Dosierungen 34 bezüglich der eingesetzten Strahlung oder der eingesetzten medizinischen Mittel oder auch die Standorte 36 der genutzten medizinischen Modalitäten 12 gespeichert. Es ist ferner denkbar, dass die medizinischen Bilddaten 16 bzw. die medizinischen Bilddatensätze 17 auf einem Zentralrechner 22 gespeichert werden und die unterschiedlichen interaktiven Betrachter 24, insbesondere mittels Internetverbindung oder mittels VPN-Verbindung, direkt auf den Zentralrechner 22 zugreifen können, um an die medizinischen Bilddaten 16 bzw. die medizinischen Bilddatensätze 17 zu gelangen. Diese können die unterschiedlichen Betrachter 24 dann auf ihrem Anzeigemedium 18 (s. Fig. 3) darstellen lassen. Dies kann beispielsweise mittels eines 3D-fähigen Clienten (wie oben bereit beschrieben), welcher als virtueller oder realer Client vorliegen kann, erfolgen.

Figur 3 zeigt ein Anzeigemedium 18 eines interaktiven Betrachters 24, wobei der Betrachter insbesondere eine 3D-Brille verwendet, um die medizinischen Bilddaten 16, 16a als dreidimensionale Struktur in Virtual Reality sehen zu können. Auf dem Anzeigemedium 18 ist ein Untersuchungsbereich 30 eines Patienten 14 in einer virtuellen Umgebung 42 bzw. in einer erweiterten Realität 44 dargestellt. In Figur 3 ist beispielsweise als Untersuchungsbereich 30 ein Gehirn eines Patienten 14 dargestellt. In der erweiterten Realität 44 sind nicht ortskodierte Daten 46 wie beispielsweise Patienteninformationen oder Labordaten etc. gespeichert. Ferner ist in der erweiterten Realität 44 wie in Figur 3 gezeigt mindestens ein Zeiger 54 mit einem Hyperlink 50 hinterlegt, in welchen Notizen des entsprechenden interaktiven Betrachters hinterlegt sind.

Jedem der interaktiven Betrachter 24 werden auf einem ihm zugeordneten Anzeigemedium 18 die medizinischen Bilddaten 16 dargestellt. Ferner ist es denkbar, dass mehreren der interaktiven Betrachtern zusammen auf einem Anzeigemedium 18 die medizinischen Bilddaten 16 dargestellt werden. Jedem der interaktiven Betrachter 24 ist in der virtuellen Umgebung 42 und/oder der erweiterten Realität 44 eine Grafikfigur 48 zugeordnet. Die Position der Grafikfigur 48 gibt die Position bzw. den Blickwinkel des jeweiligen interaktiven Betrachters 24 in der virtuellen Umgebung 42 ins bzw. auf den Untersuchungsbereich 30 wieder.

Hierdurch wird in der virtuellen Umgebung die Position bzw. der Blickwinkel eines interaktiven Betrachters 24 den anderen interaktiven Betrachtern 24 mitgeteilt. Aufgrund der Positionen der Grafikfiguren 48 wird dem ersten interaktiven Betrachter 24 der Gruppe aus interaktiven Betrachtern 24 die virtuelle Position der jeweiligen anderen interaktiven Betrachtern 24 in der Darstellung der medizinischen Bilddaten mitgeteilt. Beispielsweise kann der erste interaktive Betrachter 24 bei Bedarf seine ihm zugeordnete Grafikfigur, insbesondere mittels eines Bedienelementes 52, wie einer virtuellen oder realen 3D-Maus, einblenden lassen. Hierdurch kann der erste interaktive Betrachter seine eigne virtuelle Position bzw. seinen Blickwinkel mit den Positionen bzw. Blickwinkeln der anderen interaktiven Betrachtern 24 vergleichen bzw. abgleichen.

Weiter bevorzugt kann jeder Betrachter 24 die Position und/oder den Blickwinkel eines anderen Betrachters 24 einnehmen, oder ein Betrachter 24 kann veranlassen, dass ein anderer Betrachter 24 in der virtuellen Umgebung 42 seine Position und/oder seinen Blickwinkel einnimmt. Hierdurch kann ein Betrachter 24 oder können mehrere Betrachter 24 einfach und schnell die Position bzw. den Blickwinkel eines anderen Betrachters 24 einnehmen, um dessen Analyse bzw. Bewertung der medizinischen Bilddaten 16 einfacher nachvollziehen zu können. Es ist denkbar, dass ein Betrachter 24 die anderen Betrachter 24 veranlasst seine Position einzunehmen, ohne dass die anderen Betrachter 24 eine Position wählen können. In einem solchen Fall sehen die Betrachter 24 die Darstellung der veränderten medizinischen Bilddaten 16a des jeweiligen Betrachters 24.

Ein solches Umschalten (nicht gezeigt) von einer Position eines Betrachters 24 auf eine andere Position, insbesondere auf eine Position eines anderen Betrachters 24, erfolgt mittels eines allmählichen Perspektivwechsels. Bei einem allmählichen Perspektivwechsel werden weiche Trajektorien der interaktiven Betrachter 24 in der virtuellen Umgebung 42 berechnet. Für die Berechnung der weichen Trajektorien wird die Position der einem jeden der interaktiven Betrachter 24 zugeordneten Grafikfigur 48 in der virtuellen Umgebung 42 berücksichtigt. Hierdurch kann ein abrupter Perspektivenwechsel vermieden werden. Ferner werden hierdurch allen oder ausgewählten Betrachtern 24 gleichzeitig und in Echtzeit die veränderten medizinischen Bilddaten 16a eines Betrachters 24 dargestellt. Die medizinischen Bilddaten 16 bzw. die veränderten medizinischen Bilddaten 16a werden auf Grundlage mindestens zweier medizinischer Bilddatensätze 17 bereitgestellt.

Jeder Betrachter 24 kann sich mittels eines virtuellen Bedienelementes 52 in der virtuellen Umgebung 42 bewegen und die medizinischen Bilddaten 16, 16a bzw. den mindestens einen medizinischen Bilddatensatz 17 verändern. Das virtuelle Bedienelement ist in der virtuellen Umgebung 42 angeordnet und ist für einen Betrachter 24 auf seinem Anzeigemedium 18 sichtbar. Das virtuelle Bedienelement 52 kann in der virtuellen Umgebung 42 beispielsweise als 3D-Maus oder Joystick oder als virtuelle Tastatur oder als virtuelle Konsole, insbesondere neben einem Patientendatensatz, welcher in den nichtortkodierten Daten 46 umfasst sein kann, abgebildet sein. Mit dem virtuellen Bedienelement 52 kann ein interaktiver Betrachter 24 jedenfalls die virtuelle Umgebung 42 verändern, nämlich beispielsweise durch Hinzufügen eines Zeigers 54 oder durch Segmentieren der medizinischen Bilddaten 16, 16a etc.

## Patentansprüche

1. Verfahren zur Darstellung von medizinischen Bilddaten (16, 16a)auf mindestens einem Anzeigemedium (18) für eine Gruppe aus mindestens zwei interaktiven Betrachtern (24), mit den Schritten
- Bereitstellen von medizinischen Bilddaten (16, 16a), welche zumindest einen drei- oder vierdimensionalen Bilddatensatz (17) eines bestimmten Untersuchungsbereichs (30) eines Patienten (14) enthalten;
- Bereitstellen einer Möglichkeit zur perspektivischen Darstellung der medizinischen Bilddaten (16, 16a) in einer interaktiven, virtuellen Umgebung (42), insbesondere mit erweiterter Realität (44);
- wobei jedem interaktiven Betrachter (24) eine eigene virtuelle Position und ggf. ein eigener Blickwinkel in der virtuellen Umgebung (42) zugeordnet ist; und
- wobei jedem der interaktiven Betrachter (24) gleichzeitig die medizinische Bilddaten (16, 16a) aus der dem jeweiligen Betrachter (24) zugeordneten virtuellen Position und ggf. Blickwinkel auf dem mindestens einen Anzeigemedium (18) dargestellt wird, und
- wobei jeder interaktiver Betrachter (24) seine virtuelle Position und ggf. Blickwinkel unabhängig von den anderen Betrachten (24) verändern kann.

2. Verfahren nach Anspruch 1, wobei für zumindest einen, vorzugsweise alle, interaktiven Betrachter (24) eine Möglichkeit zur Bearbeitung der medizinischen Bilddaten (16, 16a) und/oder zur Veränderung der Darstellung der medizinischen Bilddaten (16, 16a) bereitgestellt wird.

3. Verfahren nach Anspruch 2, wobei jeder Betrachter (24) auswählen kann, ob nach einer Bearbeitung der medizinischen Bilddaten (16, 16a) und/oder Veränderung der Darstellung der medizinischen Bilddaten (16, 16a) den anderen Betrachtern (24) oder einem bestimmten anderen Betrachter (24) in Echtzeit des zumindest einen bearbeiteten medizinische Bilddatensatzes (17) bzw. die veränderte Darstellung auf dem mindestens einen Anzeigemedium (18) dargestellt wird.

4. Verfahren nach Anspruch 2 oder 3, wobei die Bearbeitung der medizinischen Bilddaten (16, 16a) bzw. die Veränderung der Darstellung der medizinischen Bilddaten (16, 16a) mindestens eine der folgenden Aktionen umfasst:
- Anbringen eines virtuellen Zeigers (54), insbesondere wahlweise mit einem hinterlegtem Hyperlink (50) auf einen eingegebenen Text und/oder Grafik und/oder einer Audiospur an einer bestimmten Position eines medizinischen Bilddatensatzes,
- Auswählen einer Segmentierung eines medizinischen Bilddatensatzes (17), welche beispielsweise nur Knochen und/oder nur Knorpel und/oder nur Gefäße des Untersuchungsbereiches zeigt,
- Umschalten zwischen verschiedenen medizinischen Bilddatensätzen (17) desselben Untersuchungsbereichs (30),
- Entfernen und/oder Schwärzen von Teilen eines dreidimensionalen medizinischen Bilddatensatzes (17),
- Überlagern eines ersten dreidimensionalen medizinischen Bilddatensatzes (17) des Untersuchungsbereichs (30) mit einem zweiten dreidimensionalen medizinischen Bilddatensatz (17) des Untersuchungsbereichs (30), welche insbesondere mit unterschiedlichen Modalitäten (12) oder Gewichtungen oder zu unterschiedlichen Zeitpunkten aufgenommen wurden,
- Einblenden von nicht-ortskodierten Daten (46) des Patienten (14) in die virtuelle Umgebung (42),
- im Fall von einem vierdimensionalen medizinischen Bilddatensatz (17), welcher in einer Schleife als 3D-Film dargestellt wird, Anhalten des 3D-Films an einer beliebigen zeitlichen Position,
- Anzeigen eines Laborwertes zu einem dargestellten Organ im Untersuchungsbereich (30) bei einem Anklicken dieses Organes im virtuellen Raum,
- Einblenden funktioneller medizinischer Daten wie beispielsweise medizinische Daten der funktionellen MRT oder der funktionellen CT.

5. Verfahren nach einem der vorherigen Ansprüche, wobei die medizinische Bildinformation für die Gruppe aus Betrachtern (24) jeweils auf demselben Anzeigemedium (18) oder auf einem für einen Teil der Gruppe aus Betrachtern (24) jeweils eigenem Anzeigemedium (18) dargestellt wird.

6. Verfahren nach einem der vorherigen Ansprüche, wobei die medizinischen Bilddaten (16, 16a) mindestens zwei drei- oder vierdimensionale medizinische Bilddatensätze (17) desselben Untersuchungsbereiches (30), sowie insbesondere weitere nicht-ortskodierte Daten (46) des Patienten (14) enthalten.

7. Verfahren nach einem der vorherigen Ansprüche, wobei die medizinischen Bilddaten (16, 16a) auf einem Zentralrechner (22) oder einer Cloud (26) bereitgestellt werden.

8. Verfahren nach einem der vorhergehenden Ansprüche, wobei die virtuelle, interaktive Umgebung (42) für jeden Betrachter (24) durch einen 3D-fähigen virtuellen oder realen Client aufgebaut wird.

9. Verfahren nach einem der vorherigen Ansprüche, wobei in der virtuellen Umgebung (42) einem ersten interaktiven Betrachter (24) mindestens eine Grafikfigur (48) an einer Position dargestellt wird, welcher der virtuellen Position eines anderen Betrachters (24) in der virtuellen Umgebung (42) entspricht, wobei aufgrund der Position der Grafikfigur (48) dem ersten Betrachter (24) der Gruppe die virtuelle Position des jeweiligen anderen Betrachters (24) in der Darstellung der medizinischen Bilddaten (16, 16a) mitgeteilt wird.

10. Verfahren nach einem der vorherigen Ansprüche, wobei jeder Betrachter (24) die Position und/oder den Blickwinkel eines anderen Betrachters (24) einnehmen kann, oder wobei ein Betrachter (24) veranlassen kann, dass ein anderer Betrachter (24) in der virtuellen Umgebung (42) seine Position und/oder seinen Blickwinkel einnimmt.

11. Verfahren nach einem der vorherigen Ansprüche, wobei ein Umschalten von einer Position eines Betrachters (24) auf eine andere Position, insbesondere auf eine Position eines anderen Betrachters (24), mittels eines allmählichen Perspektivwechsels erfolgt.

12. Verfahren nach einem der vorherigen Ansprüche, wobei für jeden Betrachter (24) ein virtuelles Bedienelement (52) in der virtuellen Umgebung (42) angeordnet ist.

13. Computerprogramm, enthaltend Softwarecode-Abschnitte, wobei die Softwarecode-Abschnitte einen Computer oder ein Netzwerk aus Rechnern oder ein Endgerät in einem Netzwerk, welches mit einem Zentralrechner (22) kommuniziert, dazu veranlassen, ein Verfahren nach einem der Ansprüche 1 bis 12 auszuführen.

14. Vorrichtung zur Darstellung von medizinischen Bilddaten (16, 16a) auf mindestens einem Anzeigemedium (18) für eine Gruppe aus mindestens zwei interaktiven Betrachtern (24), wobei die Vorrichtung insbesondere zur Durchführung des Verfahrens nach einem der Ansprüche 1 bis 13 konfiguriert ist, umfassend
- mindestens ein Speichermedium zum Hinterlegen der medizinischen Bilddaten (16, 16a), wobei die Bilddaten (16, 16a) mindestens einen drei- oder vierdimensionale Bilddatensatz (17) eines bestimmten Untersuchungsbereichs (30) eines Patienten (14) enthalten,
- mindesten ein Anzeigemedium (18), welches für eine perspektivische dreidimensionale Darstellung der medizinischen Bilddaten (16, 16a) geeignet ist,
- eine Recheneinheit, welche dazu konfiguriert ist, die medizinischen Bilddaten (16, 16a) für einen interaktiven Betrachter (24) in einer interaktiven, virtuellen Umgebung (42) perspektivisch darzustellen, insbesondere mit erweiterter Realität (44);
wobei jedem interaktiven Betrachter (24) eine eigene virtuelle Position und ggf. ein eigener Blickwinkel in der virtuellen Umgebung (42) zugeordnet ist; und
wobei jedem der interaktiven Betrachter (24) gleichzeitig die medizinische Bilddaten (16, 16a) aus der dem jeweiligen Betrachter (24) zugeordneten virtuellen Position und ggf. Blickwinkel auf dem mindestens einen Anzeigemedium (18) dargestellt werden, und
- wobei jeder interaktiver Betrachter (24) seine virtuelle Position und ggf. Blickwinkel unabhängig von den anderen Betrachtern (24) verändern kann.

15. Vorrichtung nach Anspruch 14, ferner umfassend
- eine dreidimensionale Maus, und/oder
- eine 3D-Brille für jeden Betrachter.
